# EUROPÄISCHE PATENTANMELDUNG

(11) **EP 0 750 185 A2**
(43) Veröffentlichungstag der Anmeldung: **27.12.1996**
(21) Anmeldenummer: 96109902.5
(22) Anmeldetag: 20.06.1996
(51) Int. Cl.: G01N 1/28

(54) **Element und System zum Sammeln, Transportieren und Lagern von zu analysierendem Probenmaterial**

(30) Priorität: 24.06.1995 DE 19523061
(71) Anmelder: BOEHRINGER MANNHEIM GMBH, 68298 Mannheim (DE)
(72) Erfinder: Wielinger, Hans, Dr., 69469 Weinheim (DE); Lerch, Rolf, 68549 Ilvesheim (DE); Werner, Wolfgang, Dr., 68309 Mannheim (DE)

(57) **Zusammenfassung**

Gegenstand der Erfindung ist ein Element zum Sammeln, Transportieren und Lagern von zu analysierendem Probenmaterial enthaltend saugfähiges Matrixmaterial,
dadurch gekennzeichnet, daß
das Element eine erste und eine zweite Schicht saugfähigen Matrixmaterials so sich berührend nebeneinander auf einem inerten Trägermaterial in einem Flüssigkeitsübertritt ermöglichenden Kontakt angeordnet enthält, daß Flüssigkeit von der ersten in die zweite Schicht gelangen kann, wenn die erste Schicht mit Flüssigkeit gefüllt ist und
die erste Schicht nach Aufbringen und Eintrocknen des Probenmaterials vollständig von der zweiten Schicht abgetrennt werden kann
sowie ein System enthaltend ein solches Element und eine verschließbare Umhüllung, in der das Element transportiert werden kann.

Gegenstand der Erfindung ist auch ein Verfahren zur Analyse flüssigen Probenmaterials, bei dem flüssiges Probenmaterial auf ein erfindungsgemäßes Element gegeben, zur Analyse die erste von der zweiten Schicht saugfähigen Matrixmaterials getrennt und anschließend eluiert wird.

## Beschreibung

Die Erfindung betrifft ein Element zum Sammeln, Transportieren und Lagern von zu analysierendem Probenmaterial enthaltend saugfähiges Matrixmaterial. Die Erfindung betrifft weiter ein System enthaltend ein solches Element und eine verschließbare Umhüllung, in der das Element transportiert werden kann. Außerdem betrifft die Erfindung ein Verfahren zur Analyse flüssigen Probenmaterials, bei dem dieses auf ein saugfähiges Matrixmaterial aufgegeben, dort eingetrocknet und zu einer Analysestation transportiert wird, zur Analyse aus dem Matrixmaterial und der Bildung einer Lösung eluiert und diese Lösung analysiert wird. Der Transport von Probenmaterial von Patienten in ein Labor ist ein wesentlicher Schritt bei der Gewinnung eines Analysenergebnisses. Beruhend auf dem Analysenergebnis stellt der Arzt eine Diagnose und beurteilt so beispielsweise die Stoffwechsellage von Patienten mit Diabetes, Fettstoffwechselstörungen, hormonellen Störungen usw. Meistens werden Körperflüssigkeiten direkt beim behandelnden Arzt analysiert oder von dort in Transportgefäßen in ein Labor geschickt. Der Transport solcher Proben ist aufwendig und mit Risiken, wie zum Beispiel Bruch des Transportgefäßes verbunden. Auch wenn nur kleine Probenmengen für eine Analyse gebraucht werden, müssen dennoch für einen solchen Transport oft größere Mengen entnommen werden. So ist oft eine Venenpunktion notwendig, wenn Blut in einem Labor untersucht werden soll, obwohl eine einfache Abnahme von Kapillarblut zum Beispiel aus der Fingerbeere ausreichte.

Aus diesen Gründen fehlte es nicht an Bemühungen, insbesondere für die quantitative Bestimmung von Substanzen, die aus wenig Probenmaterial möglich ist, Abnahme und Transportsysteme für Kapillarblut zu entwickeln, wie zum Beispiel das Unisept®-System der Firma Owen Mumford Ltd., Oxford, Großbritannien. Dieses System ist beispielsweise in Scand. J. Clin. Lab. Invest. 46, 315-317 (1986) beschrieben. Hiermit kann Kapillarblut als solches zur anschließenden Bestimmung von HbA_{lc} gewonnen, flüssig gelagert und zum Analyseort transportiert werden. Es ist einsehbar, daß der Transport solcher Flüssigkeitsbehältnisse aufwendig ist und ein erhebliches Bruch- und damit auch Infektionsrisiko impliziert.

Eine Methode auch kleinere Mengen an Probenmaterial zum Analyseort zu transportieren, wird beispielsweise in Br. Med. J. 2, 468-469 (1978) vorgestellt. Zur Bestimmung von Glukose wird Kapillarblut auf Filterpapier aufgegeben und dort eintrocknen gelassen. Dieses Filterpapier wird so zum Ort der Untersuchung transportiert. Dort wird aus dem Filterpapier eine Scheibe mit Probe ausgestochen, eluiert und das Eluat untersucht. Ungenauigkeiten ergeben sich hierbei durch Größenvariationen der ausgestochenen Scheiben und durch unterschiedliche Spreitverhalten der aufgetragenen Blutproben, die mit dem unterschiedlichen Gehalt an Blutzellen in der Probe erklärt werden. Das Erfordernis des Ausstechens einer Papierscheibe aus dem Filterpapier bedeutet auch bei dieser Methode einen aufwendigen Umgang mit potentiell infektiösen Material.

Aufgabe der vorliegenden Erfindung ist es deshalb gewesen, ein Element zum Sammeln, Transportieren und Lagern von zu analysierendem Probenmaterial enthaltend saugfähiges Matrixmaterial zur Verfügung zu stellen, das den Umgang mit eingetrockneten Probenflüssigkeiten erleichtert und eine möglichst homogene Verteilung der Probe gewährleistet.

Diese Aufgabe wird durch die in den Patentansprüchen näher charakterisierte Erfindung gelöst.

Gegenstand der Erfindung ist ein Element zum Sammeln, Transportieren und Lagern von zu analysierendem Probenmaterial enthaltend saugfähiges Matrixmaterial, dadurch gekennzeichnet, daß das Element eine erste und eine zweite Schicht saugfähigen Matrixmaterials so sich berührend nebeneinander auf einem inerten Trägermaterial in einem Flüssigkeitsübertritt ermöglichenden Kontakt angeordnet enthält, daß Flüssigkeit von der ersten in die zweite Schicht gelangen kann, wenn die erste Schicht mit Flüssigkeit gefüllt ist und die erste Schicht nach Aufbringen und Eintrocknen des Probenmaterials vollständig von der zweiten Schicht abgetrennt werden kann.

Gegenstand der Erfindung ist auch ein System enthaltend ein erfindungsgemäßes Element und eine verschließbare Umhüllung, in der das Element transportiert werden kann.

Außerdem ist Gegenstand der Erfindung ein Verfahren zur Analyse flüssigen Probenmaterials, bei dem flüssiges Probenmaterial auf ein saugfähiges Matrixmaterial aufgegeben, dort eingetrocknet und zu einer Analysestation transportiert wird, zur Analyse aus dem Matrixmaterial unter Bildung einer Lösung eluiert und diese Lösung analysiert wird, dadurch gekennzeichnet, daß das flüssige Probenmaterial auf die erste Schicht saugfähigen Matrixmaterials eines Elementes gemäß einem der Patentansprüche 1-6 gegeben wird und zur Analyse die erste von der zweiten Schicht saugfähigen Matrixmaterials getrennt und anschließend eluiert wird.

Das erfindungsgemäße Element ist zum Sammeln, Transportieren und Lagern von zu analysierendem Probenmaterial, insbesondere flüssigen Proben, vor allem Körperflüssigkeiten wie Blut, Plasma, Serum, Urin, Speichel etc. geeignet. Besonders bevorzugt wird das erfindungsgemäße Element zum Sammeln, Transportieren und Lagern von Blutproben eingesetzt. Das erfindungsgemäße Element besitzt auf einem inerten Trägermaterial zwei Schichten saugfähigen Matrixmaterials so sich berührend nebeneinander angeordnet, daß ein Flüssigkeitsübertritt von der ersten in die zweite Schicht möglich ist, wenn die erste Schicht mit Flüssigkeit gefüllt ist.

Als saugfähige Matrixmaterialien werden bevorzugt faserige Materialien eingesetzt, obwohl grundsätzlich auch nicht-faserige Materialien, wie beispielsweise Membranen, einsetzbar sind. Bevorzugte faserige saugfähige Matrixmaterialien sind Vliese, Gewebe oder Gewirke. Vliese sind ganz besonders bevorzugt. Die faserigen Matrixmaterialien können Glas-, Zellulose-, Polyesterfasern aber auch Viskose und Polyvinylalkohol enthalten. Vliesmaterialien, enthaltend schmelzbare Copolyesterfasern neben Glasfasern, Polyesterfasern Polyamidfasern, Zellulosefasern oder Zellulosederivatefasern, wie sie in der europäischen Patentanmeldung 0 571 941 beschrieben sind, können ebenfalls vorteilhaft in dem erfindungsgemäßen Element eingesetzt werden.

Je nach dem zu analysierenden Analyt muß sichergestellt werden, daß dieser nach Aufgabe und Eintrocknen des Probenmaterials auf das saugfähige Matrixmaterial insbesondere das Matrixmaterial der ersten Schicht anschließend wieder reproduzierbar eluiert werden kann. Hierfür kann der Fachmann einfache Elutionsversuche durchführen, um sich Gewißheit zu verschaffen.

Als eine wichtige Eigenschaft der erfindungsgemäß einsetzbaren saugfähigen Matrixmaterialien ist deren Saugfähigkeit zu beachten. Erfindungsgemäß soll die Saugfähigkeit des Matrixmaterials der ersten Schicht gleich groß oder größer sein als die der zweiten benachbarten Schicht. Dadurch wird vermieden, daß störende Saugeffekte ausgebildet werden, wenn Probenmaterial auf die erste Schicht aufgebracht wird.

Die Saugfähigkeit kann nach DIN 53106 bestimmt werden. Hierzu werden Proben von 200+/-1 mm Länge und 15+/-0,1 mm Breite lotrecht mit ihrem unteren Ende 25 mm in destilliertem Wasser eingetaucht und der Weg den das Wasser in 10 Minuten zurücklegt in mm gemessen.

Wie unterschiedliche Saugfähigkeiten bei Matrixmaterialien gleicher Bestandteile eingestellt werden können, ist dem Fachmann bekannt. Beispielsweise können bei der Herstellung von Vliesen Fasern unterschiedlicher Dicke verwendet werden. Je dicker die eingesetzten Fasern sind, desto geringer ist die Saugfähigkeit. Ein weiterer Weg ist die Variation der Dichte von Vliesen. Durch eine Erhöhung der Dichte wird die Saugfähigkeit reduziert.

Bei der Verwendung von Geweben haben Gewebe mit feineren Fasern eine größere Saugfähigkeit als Gewebe mit gröberen Fasern. Aber auch über unterschiedliche Arten der Verzwirnung der Fäden kann die Saugfähigkeit gesteuert werden. Ebenso können über die Webarten Unterschiede in der Saugfähigkeit erzielt werden. Weitere Variationsmöglichkeiten der Saugfähigkeit können durch die Verwendung von unterschiedlichen Fasermischungen erreicht werden. So wird zum Beispiel durch den Zusatz von hydrophoben Fasern die Saugfähigkeit verringert.

Als inertes Trägermaterial für die beiden erfindungsgemäß einsetzbaren Matrixmaterialschichten kommen insbesondere steife Materialien in Frage, wie beispielsweise Kunststoffolien, Karton, beschichtetes Papier usw.

Auf dem inerten Trägermaterial sind die Matrixmaterialschichten so befestigt, daß die Flüssigkeitsaufnahme durch die Matrixmaterialien nicht beeinträchtigt wird. Dies ist durch Verwendung eines doppelseitig klebenden Bandes oder beispielsweise auch durch die Verwendung von Schmelzkleber möglich.

Die Matrixmaterialschichten müssen auf dem inerten Trägermaterial so befestigt sein, daß die erste Schicht nach Aufbringen und Eintrocknen flüssigen Probenmaterials vollständig von der zweiten Schicht abgetrennt werden kann. Dies ist insbesondere dann möglich, wenn die erste Schicht nur relativ lose oder nur punktuell befestigt ist.

Die beiden Matrixmaterialschichten müssen so nebeneinander sich berührend auf dem Trägermaterial angeordnet sein, daß ein Flüssigkeitsübertritt von der ersten Schicht in die zweite Schicht möglich ist, wenn die erste Schicht mit Flüssigkeit gefüllt ist. Dies ist dann möglich, wenn zumindest eine Kantenberührung der beiden Schichten vorliegt. Noch besser ist jedoch eine leichte Überlappung der beiden Schichten vorgesehen. Besonders bevorzugt sind die Schichten dann so angeordnet, daß die zweite Schicht die erste Schicht leicht überlappt.

Die Größe der Matrixmaterialschichten muß so gewählt werden, daß die erste Schicht, die später auch als Auswerteschicht verwendet wird, mit der Probenflüssigkeit vollständig gefüllt werden kann. Überschüssige Probenflüssigkeit wird dann von der zweiten Schicht aufgenommen. Welche Probenmengen ausreichend sind zur Bestimmung eines bestimmten Analyten hängt von der Art des zu bestimmenden Analyten ab. In der Regel reichen jedoch 5-20 µl, meistens 10 µl Probe aus. Dieses Volumen muß von der ersten Matrixschicht aufgenommen und später wieder eluiert werden können. Zur Sicherheit sollte die zweite Matrixschicht, die die Funktion einer Saugschicht darstellt, ein größeres Volumen aufnehmen können. Saugvolumen von 10-50, bevorzugt 10-30 µl, besonders bevorzugt 20 µl reichen hierzu in der Regel aus. Zweckmäßigerweise sollten die üblichen Abmessungen der Matrixmaterialschichten so beschaffen sein, daß das Saugvolumen der beiden Matrixmaterialschichten zusammengenommen mindestens 30 µl, besser mindestens 50 µl beträgt. Über eine solche Dimensionierung wird gewährleistet, daß sowohl mit kleinen als auch mit großen Flüssigkeitstropfen auf die erste Matrixschicht verschiedener erfindungsgemäßer Elemente die gleiche Menge an Probe aufgebracht wird. Die kleinere erste Schicht hat zur Erreichung eines ausreichenden Saugvolumens in der Regel eine Fläche von 3x3 bis 8x8 mm.

Durch die vorbeschriebene Anordnung der Matrixmaterialschichten wird eine homogene Verteilung flüssigen Probenmaterials in der ersten Schicht erreicht. Dadurch, daß die erste Schicht vollständig mit flüssigem Probenmaterial gefüllt werden soll, können sich innerhalb dieser Schicht keine Konzentrationsgradienten des Analyten ausbilden, die in den Randzonen der Elemente des Standes der Technik sonst immer zu beobachten waren. Konzentrationsbedingte Meßunterschiede bei der Bestimmung von Analyten werden so vermieden.

Um eine Trennung der ersten von der zweiten Schicht saugfähigen Matrixmaterials im erfindungsgemäßen Element sicherzustellen, sind verschiedene Anordnungen der Schichten auf dem Trägermaterial denkbar. Bevorzugte Ausführungsformen des erfindungsgemäßen Elementes sind in Figuren 1 und 2 dargestellt. Figur 3 zeigt ein System enthaltend ein erfindungsgemäßes Element und eine verschließbare Umhüllung, in der das Element transportiert werden kann.

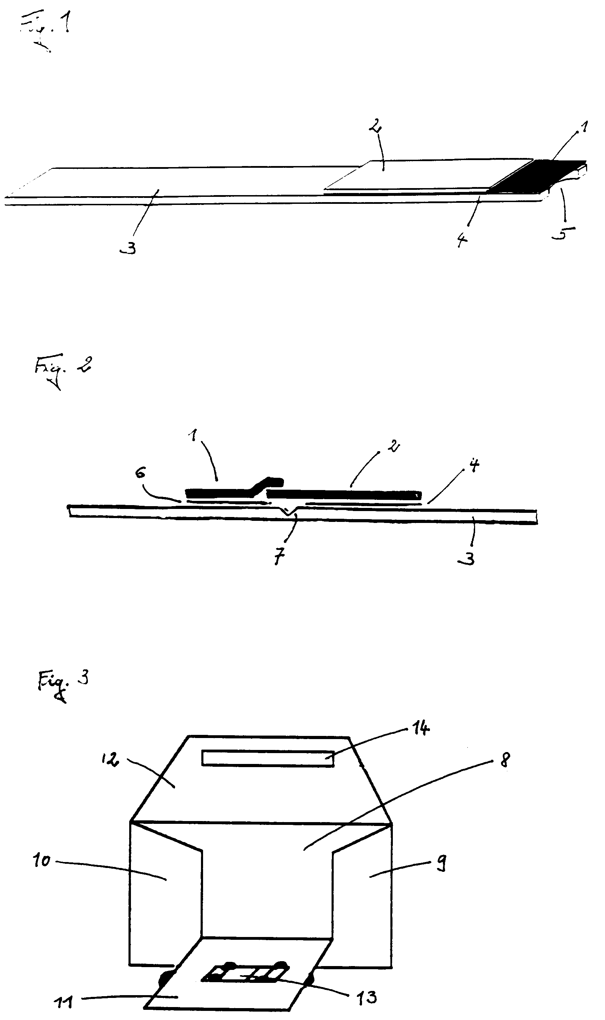

Das erfindungsgemäße Element gemäß Figur 1 trägt saugfähige Matrixmaterialschichten (1, 2) an einem Ende eines inerten Trägermaterials (3). Die Schichten sind mittels eines doppelseitig klebenden Bandes (4) auf dem Trägermaterial (3) befestigt. Die Schichten (1, 2) sind so auf dem Trägermaterial (3) angeordnet, daß sie sich am Ende dieses Trägermaterials (3) befinden. Die erste saugfähige Matrixmaterialschicht (1), die für den Probenauftrag vorgesehen ist, liegt dem Ende des Trägermaterials (3) am nächsten. Sie wird leicht überlappt durch die zweite saugfähige Matrixmaterialschicht (2), die überschüssige Flüssigkeit des Probenmaterials aufnimmt, wenn die erste Schicht (1) gefüllt ist. Am Ende des Trägermaterials (3) unterhalb der ersten Schicht (1) befindet sich eine Aussparung (5) in dem Trägermaterial (3). Diese Aussparung (5) ermöglicht bzw. erleichtert das Greifen der ersten Schicht (1), beispielsweise mit einer Pinzette, um diese aus dem Element zu entfernen.

Für das erfindungsgemäße Verfahren zur Analyse flüssigen Probenmaterials wird dieses auf die erste Schicht saugfähigen Matrixmaterials (1) gegeben. Vorzugsweise wird soviel flüssiges Probenmaterial aufgegeben, daß die erste Schicht (1) vollständig vollgesaugt ist. Besonders bevorzugt wird soviel flüssiges Probenmaterial aufgegeben, daß das Saugvolumen der ersten Schicht (1) nicht ausreicht, die Flüssigkeit vollständig aufzunehmen. Der Überschuß an Flüssigkeit gelangt über die erste Schicht (1) in die zweite Schicht saugfähigen Matrixmaterials (2). Das zu untersuchende flüssige Probenmaterial trocknet auf dem erfindungsgemäßen Element ein und wird so zu einer Analysestation gegeben. Der Probenauftrag kann durch Patienten selbst oder auch durch einen behandelnden Arzt vorgenommen werden. Die Analyse wird in der Regel in einem Labor durchgeführt. Zur Analyse des auf dem Element befindlichen Probenmaterials wird die erste Schicht saugfähigen Matrixmaterials (1) aus dem Element entfernt. Im Falle des erfindungsgemäßen Elementes gemäß Figur 1 kann dies beispielsweise mittels einer Pinzette geschehen, die im Bereich der Aussparung (5) die erste Schicht (1) greift und aus dem Element entfernt. In diesem Zusammenhang ist es ersichtlich, daß zur vollständigen Entfernung der ersten Schicht (1) des Elementes diese nur so fest an dem Trägermaterial (3) fixiert sein darf, daß diese vollständige Entfernung auch möglich ist. Im vorliegenden Fall ist dies dadurch sichergestellt, daß Schicht (1) nur über einen schmalen Streifen doppelseitigen Klebebandes (4) an das inerte Trägermaterial (3) fixiert ist, während die zweite Matrixmaterialschicht (2) über eine wesentlich größere Fläche mit doppelseitig klebendem Band (4) befestigt ist. Durch die vollständige Entfernbarkeit der ersten Schicht (1) wird sichergestellt, daß immer gleich große Probenmengen zur Untersuchung gelangen. Die aus dem Element herausgetrennte Schicht (1) wird eluiert, um den zu bestimmenden Analyt in Lösung zu bringen. Ja nach Analyt werden hierzu dem Fachmann bekannte Elutionsmittel eingesetzt. Die Analyse der resultierenden Lösungen erfolgt je nach zu bestimmenden Analyt ebenfalls nach dem Fachmann bekannten Methoden.

In dem erfindungsgemäßen Element gemäß Figur 2 sind die beiden Schichten saugfähigen Matrixmaterials (1, 2) so auf dem inerten Trägermaterial (3) befestigt, daß zwei gegenüberliegende Enden des Trägermaterials (3) frei und mit den Fingern greifbar sind. Die beiden Matrixmaterialschichten (1,2) sind mittels doppelseitig klebendem Band (4,6) auf dem Trägermaterial (3) befestigt. Das Trägermaterial (3) besitzt eine Sollbruchstelle (7), die so angeordnet ist, daß beim Biegen, Brechen oder Reißen das Element dort so in zwei Teile geteilt werden kann, daß der eine die erste Schicht saugfähigen Matrixmaterials (1) und der andere die zweite Schicht saugfähigen Matrixmaterials (2) trägt. Im Falle einer Plastikfolie als Trägermaterial (3) kann die Sollbruchstelle (7) eine Einkerbung sein. Es kann dort jedoch auch eine entsprechende Perforierung vorliegen, die es ermöglicht, durch Biegen des Elementes an dieser Stelle zwei getrennte Teile zu erhalten.

Es wird so viel flüssiges Probenmaterial auf die erste Matrixmaterialschicht (1) gegeben, daß diese Schicht (1) vollständig mit Flüssigkeit gefüllt wird. Durch die Überlappung der ersten Schicht (1) mit der zweiten Schicht (2) wird sichergestellt, daß überschüssige Flüssigkeit in die zweite Schicht (2) gelangt. An der Analysestation, an der die Analyse des Probenmaterials vorgenommen werden soll, wird das Element an der Sollbruchstelle durch Biegen, Brechen oder Reißen geteilt. Der Teil des Elementes, der die erste Schicht saugfähigen Matrixmaterials (1) enthält, wird mit Elutionsmittel behandelt, so daß der zu bestimmende Analyt aus dem Matrixmaterial in Lösung geht. Die so erhaltene Lösung wird dann analysiert. Auf diese Art und Weise wird ein Kontakt mit dem das Probenmaterial enthaltenden Matrixmaterial vollständig vermieden und das Infektionsrisiko minimiert.

Während das erfindungsgemäße Element gemäß Figur 1 sowohl für die Untersuchung von Blut- als auch Urinproben besondere Vorteile aufweist, ist das erfindungsgemäße Element gemäß Figur 2 vor allem für Blutuntersuchungen geeignet. Für Blutuntersuchungen wird der Finger des Patienten mit einer Lanzette gestochen und der austretende Blutstropfen wird auf die Oberseite der ersten Schicht saugfähigen Matrixmaterials (1) des erfindungsgemäßen Elementes gegeben. Bei Harn als Probe kann beispielsweise die Seite des erfindungsgemäßen Elementes gemäß Figur 1, die die erste Schicht saugfähigen Matrixmaterials (1) enthält, so in den Harn eingetaucht werden, daß die zweite Schicht saugfähigen Matrixmaterials (2) nicht in den Harn selbst eintaucht.

Erforderlichenfalls können die saugfähigen Matrixmaterialschichten des erfindungsgemäßen Elementes Substanzen, beispielsweise zur Erhöhung der Benetzbarkeit oder zur Stabilisierung des zu bestimmenden Analyten, tragen. Insbesondere Substanzen zur Stabilisierung des zu bestimmenden Analyten können in der ersten Schicht des Elementes, auf das die Probe aufgegeben wird, enthalten sein.

Zum Transport des erfindungsgemäßen Elementes nach Aufgabe des Probenmaterials zur Analysestation hat es sich als zweckmäßig erwiesen, dieses innerhalb einer verschließbaren Umhüllung auf den Weg zu bringen. Als verschließbare Umhüllung eignet sich beispielsweise ein besonders gestalteter Umschlag, wie er in Figur 3 dargestellt ist. Dieser Umschlag enthält ein Vorderteil (8) und zwei Seitenteile (9, 10) sowie eine Rückenklappe (11) und eine Verschlußlasche (12). Das erfindungsgemäße Element zur Aufnahme des flüssigen Probenmaterials (13) befindet sich so auf der Rückenklappe (11) abnehmbar fixiert, daß beim Herunterklappen der Rückenklappe (11) dieses Element für die Aufgabe des flüssigen Probenmaterials zugänglich ist. Nach Aufgabe und Eintrocknen des Probenmaterials wird die Rückenklappe (11) hochgeklappt in Richtung der Seitenteile (9,10) und des Vorderteils (8). Auf der Verschlußlasche (12) befindet sich ein Klebestreifen (14), mit dem der Umschlag nach dem Herunterklappen der Verschlußlasche (12) über die Seitenteile (9,10) und die hochgeklappte Rückenklappe (11) verschlossen wird. Eine solche Umhüllung kann beispielsweise aus Papier oder Karton bestehen. Sie ermöglicht den hygienischen und sauberen Transport des Probenmaterial enthaltenden erfindungsgemäßen Elementes (13) zu dem Ort, an dem die Analyse des Probenmaterials vorgenommen werden soll. Dort wird der Umschlag geöffnet, die Rückenklappe (11) heruntergeklappt und das Probenmaterial enthaltende Element (13) abgenommen. Anschließend werden die erste und zweite Schicht saugfähigen Matrixmaterials des Probenmaterials enthaltenden erfindungsgemäßen Elementes getrennt, die erste Schicht eluiert und der in Lösung gegangene Analyt bestimmt.

Das erfindungsgemäße Element (13) kann auf der Innenseite der Rückenklappe (11) auf verschiedene Art und Weisen abnehmbar fixiert sein. Beispielsweise kann er dort durch entsprechende Klemmvorrichtungen eingeklemmt oder so auf einem doppelseitig klebenden Band befestigt sein, daß er zur Analyse des Probenmaterials von der Rückenklappe (11) des Umschlages abgenommen werden kann. Erfindungsgemäßes Element und verschließbare Umhüllung bilden ein System.

Das erfindungsgemäße Element und System sind geeignet zum Sammeln, Lagern und Transportieren von zu analysierendem Probenmaterial. Analyte, die so gesammelt, transportiert und gelagert werden können, schließen Glukose und glykosiliertes Hämoglobin (HbA_{lc}) ein. Im wesentlichen kann so jedoch jeder Analyt einer Messung zugeführt werden, der durch entsprechende Elutionsmittel in Lösung gebracht werden kann und der dann in dieser Lösung gemessen werden kann. Grundsätzlich sind dies beispielsweise alle Analyte, die auch über immunologische Testverfahren bestimmt werden können. Ohne den Kreis der möglichen Analyte beschränken zu wollen, seien an dieser Stelle auch solche Analyte genannt, die zum Nachweis von Infektionskrankheiten herangezogen werden, wie beispielsweise Virus-Antikörper oder Virus-Bestandteile für die Bestimmung von Hepatitis und HIV. Sie enthaltende Proben können so vorteilhaft zum Analyseort transportiert werden. Aufgrund der sauberen Umhüllung und der hygienischen Entfernbarkeit der Probenmaterial enthaltenden Schicht aus dem erfindungsgemäßen Element wird das Infektionsrisiko minimal gehalten. Dadurch, daß die saugfähige Matrixmaterialschicht (1) immer gleiche Probenvolumina aufnimmt, und dadurch, daß Sorge getragen ist, daß diese Schicht vollständig mit flüssigem Probenmaterial gefüllt wurde und sie außerdem vollständig und reproduzierbar von der zweiten Schicht saugfähigen Matrixmaterials getrennt werden kann, ist sichergestellt, daß reproduzierbare Ergebnisse hoher Genauigkeit bei anschließenden Analysen erhalten werden.

Der Gegenstand der Erfindung wird durch die nachfolgenden Beispiele noch näher erläutert.

### Beispiel 1

### Erfindungsgemäßes Element zur Aufnahme von Blut, das auf Glukose untersucht werden soll

Auf eine Polyesterfolie der Abmessungen 48 x 6 mm mit einer halbkreisförmigen Ausstanzung von 5 mm an einem kurzseitigen Ende wird mit Hilfe eines doppelseitigen Klebebandes eine erste Schicht saugfähigen Matrixmaterials, wie in Figur 1 dargestellt, so auf der Trägerfolie fixiert, daß sie mit einer Breite von 0,5 bis 1 mm auf das Klebeband geklebt wird. Über diese relativ schmale Fixierung wird die spätere Abnehmbarkeit positiv beeinflußt. Die zweite Schicht saugfähigen Matrixmaterials wird auf einer Breite von 5 mm oder mehr verklebt.

Für die erste Schicht saugfähigen Matixmaterials wird ein Vlies, das auf einer Papiermaschine hergestellt wurde und folgende technische Daten aufweist, verwendet:

100 Teile Zellulose, Mahlgrad 25° SR (Maß der Entwässerungsfähigkeit einer Faserstoffsuspension nach Merkblatt V/7/61 des Vereins der Zellstoff- und Papier-Chemiker und - Ingenieure, Fachausschuß für physikalische Halbstoff- und Papierprüfung) und 3 Teile Epichlorhydrinharz als Naßfestmittel, Flächengewicht: 100 g / m², Saughöhe: 56 mm (DIN 53106).

Dieses Vlies wird auf die Größe 6 x 6 mm zugeschnitten. Diese Matrix nimmt eine Flüssigkeitsmenge von 10 µl auf. Diese Menge muß für die spätere Berechnung der Glukosekonzentration bekannt sein.

Für die zweite Schicht saugfähigen Matrixmaterials wird ein gleich hergestelltes Vlies folgender technischer Daten eingesetzt:

100 Teile Zellulose, Mahlgrad 31° SR (Maß der Entwässerungsfähigkeit einer Faserstoffsuspension nach Merkblatt V/7/61 des Vereins der Zellstoff- und Papier-Chemiker und - Ingenieure, Fachausschuß für physikalische Halbstoff- und Papierprüfung) 3 Teile Epichlorhydrinharz, Flächengewicht: 100 g / m², Saughöhe: 42 mm (DIN 53106). Dieses Vlies wird auf die Größe 18 x 6 mm zugeschnitten.

Der Patient entnimmt mit diesem Element nach dem Anstechen der Fingerbeere durch einfaches Anhalten der ersten Matrixschicht an die Einstichstelle eine unbestimmte Menge Blut. Die für die spätere Analyse notwendige Menge an Blut wird von der ersten Matrixschicht aufgesaugt, die überschüssige Menge Blut wird an die zweite Schicht verzögert abgegeben. Der Patient steckt das Element in eine saubere Umhüllung und leitet es zum untersuchenden Labor weiter. Dort wird die erste Matrixschicht mit einer Pinzette abgenommen und, wie zum Beispiel in Br. Med. J. 2, 468-469 (1978) Glukose bestimmt.

### Beispiel 2

### Element zur Aufnahme von Blut, das auf Hämoglobin und glykosiliertes Hämoglobin (HbA_{lc}). untersucht werden soll

Ein Element vergleichbaren Aufbaus wie in Beispiel 1 beschrieben, wird aus folgenden Materialien als saugfähigen Matrixmaterialien hergestellt:

### 1. Schicht saugfähigen Matrixmaterials:

80 Teile Polyesterfasern (Faserdurchmesser 1,7 Dtex), 20 Teile Viskose, 20 Teile Polyvinylalkohol, Flächengewicht: 80 g / m², Saughöhe 102 mm (DIN 53106). Diese Schicht nimmt eine Flüssigkeitsmenge von 15 µl auf.

### 2. Schicht saugfähigen Matrixmaterials:

80 Teile Polyesterfasern (Faserdurchmesser 3,3 Dtex), 20 Teile Viskose, 20 Teile Polyvinylalkohol, Flächengewicht 80 g / m², Saughöhe 75 mm (DIN 53106).

Der Vorteil dieser Fasermischung ist die durch die Fasern bedingte geringere Polarität. Dadurch wird eine gute Wiederablösbarkeit des Hämoglobins und des glykosilierten Hämoglobins, die bestimmt werden sollen, erreicht.

In Abweichung von Beispiel 1 werden die beiden Schichten nicht über ein doppelseitig klebendes Band auf der Trägerfolie fixiert, sondern mittels eines dünnen Striches eines Heißsiegelklebers. Die erste Schicht wird über eine Breite von 0,5 bis 1,0 mm fixiert. Die zweite Schicht kann über eine größere Breite fixiert werden. Im vorliegenden Beispiel wurden 2 mm gewählt. Im übrigen wird so wie in Beispiel 1 beschrieben verfahren. Die Bestimmung von Hämoglobin und HbA_{lc} wird beispielsweise nach Elution und dem in Klin. Lab. 39, 1080 - 1082 (1993) beschriebenen Verfahren durchgeführt.

## Patentansprüche

1. Element zum Sammeln, Transportieren und Lagern von zu analysierendem Probenmaterial, enthaltend saugfähiges Matrixmaterial,
dadurch gekennzeichnet, daß
das Element eine erste und eine zweite Schicht saugfähigen Matrixmaterials so sich berührend nebeneinander auf einem inerten Trägermaterial in einem Flüssigkeitsübertritt ermöglichenden Kontakt angeordnet enthält, daß Flüssigkeit von der ersten in die zweite Schicht gelangen kann, wenn die erste Schicht mit Flüssigkeit gefüllt ist und die erste Schicht nach Aufbringen und Eintrocknen des Probenmaterials vollständig von der zweiten Schicht abgetrennt werden kann.

2. Element gemäß Anspruch 1, dadurch gekennzeichnet, daß
die Schichten saugfähigen Matrixmaterials an einem Ende des Trägermaterials so angeordnet sind, daß die erste Schicht am Ende des Trägermaterials liegt und das Trägermaterial an dem die Matrixmaterialschichten tragenden Ende eine Aussparung aufweist, die es ermöglicht, die erste Schicht zu greifen und aus dem Element zu entfernen.

3. Element gemäß Anspruch 1, dadurch gekennzeichnet, daß
die Schichten saugfähigen Matrixmaterials so auf dem inerten Trägermaterial befestigt sind, daß zwei gegenüberliegenden Enden des Trägermaterials frei und mit Fingern greifbar sind und das Trägermaterial eine Sollbruchstelle so angeordnet enthält, daß das Element durch Biegen dort so auseinandergebrochen werden kann, daß zwei Teile erhalten werden, von denen einer die erste und der andere die zweite Matrixmaterialschicht trägt.

4. Element gemäß einem der Ansprüche 1-3, dadurch gekennzeichnet, daß
die Saugfähigkeit der ersten Schicht saugfähigen Matrixmaterials gleich groß wie oder größer als die der zweiten Schicht saugfähigen Matrixmaterials ist.

5. Element gemäß einem der Ansprüche 1-4, dadurch gekennzeichnet, daß
die erste Schicht saugfähigen Matrixmaterials im wesentlichen aus Zellulosefasern besteht.

6. Element gemäß einem der Ansprüche 1-4, dadurch gekennzeichnet, daß
als erste Schicht saugfähigen Matrixmaterials eine solche aus Polyesterfasern, Viskose und Polyvinylalkohol eingesetzt wird.

7. System enthaltend ein Element gemäß einem der vorhergehenden Patentansprüche und eine verschließbare Umhüllung, in der das Element transportiert werden kann.

8. System gemäß Anspruch 7, dadurch gekennzeichnet, daß die Umhüllung wie ein Briefumschlag gestaltet ist und ein Vorder- und zwei Seitenteile sowie eine Rückenklappe und eine Verschlußlasche aufweist, wobei sich das Element auf der Rückenklappe der Umhüllung abnehmbar fixiert befindet.

9. Verfahren zur Analyse flüssigen Probenmaterials, bei dem flüssiges Probenmaterial auf ein saugfähiges Matrixmaterial aufgegeben, dort eingetrocknet und zu einer Analysestation transportiert wird, zur Analyse aus dem Matrixmaterial unter Bildung einer Lösung eluiert und diese Lösung analysiert wird,
dadurch gekennzeichnet, daß
das flüssige Probenmaterial auf die erste Schicht saugfähigen Matrixmaterials eines Elementes gemäß einem der Patentansprüche 1-6 gegeben wird und zur Analyse die erste von der zweiten Schicht saugfähigen Matrixmaterials getrennt und anschließend eluiert wird.
